# EUROPEAN PATENT SPECIFICATION

(11) **EP 3 914 237 B1**
(45) Date of publication and mention of the grant of the patent: **03.07.2024**
(21) Application number: 20701055.4
(22) Date of filing: 21.01.2020
(51) Int. Cl.: A61K 31/05, A61K 36/63, A61P 3/04

(54) **OLIVE FRUIT EXTRACT SUITABLE FOR REDUCING BODY-MASS INDEX AND VISCERAL FAT IN OVERWEIGHT SUBJECTS**
OLIVENFRUCHTEXTRAKT ZUR REDUZIERUNG DES BODY-MASS-INDEX UND DES VISZERALEN FETTS BEI ÜBERGEWICHTIGEN PROBANDEN
EXTRAIT DE FRUIT DE L'OLIVE PERMETTANT DE RÉDUIRE L'INDICE DE MASSE CORPORELLE ET LA GRAISSE VISCÉRALE CHEZ DES SUJETS EN SURPOIDS

(30) Priority: 24.01.2019 EP 19153612; 07.01.2020 EP 20386001
(43) Date of publication of application: 01.12.2021
(73) Proprietor: Ioulia & Irene Tseti Pharmaceutical Laboratories Industrial and Commercial S.A., 14564 Kifissia (GR); Tseti, Ioulia, 145 61 Kifissia Attica (GR)
(72) Inventor: TSETI, Ioulia, 145 61 Kifissia, Attikis (GR)
(74) Representative: Wibbelmann, Jobst
(86) International application number: PCT/EP2020/051345
(87) International publication number: WO 2020/152131

(56) References cited:
- CRESPO MARIA CARMEN ET AL: "One-week administration of hydroxytyrosol to humans does not activate Phase II enzymes", PHARMACOLOGICAL RESEARCH, ACADEMIC PRESS, LONDON, GB, vol. 95, 30 March 2015 (2015-03-30), pages 132-137, XP029611535, ISSN: 1043-6618, DOI: 10.1016/J.PHRS.2015.03.018 cited in the application & Maria Carmen Crespo ET AL: "One-week administration of hydroxytyrosol to humans does not activate Phase II enzymes - Supplementary data", Pharmacological Research 95 (13892), 30 March 2015 (2015-03-30), pages 1-2, XP055607094, Retrieved from the Internet: URL:https://ars.els-cdn.com/content/image/ 1-s2.0-S1043661815000572-mmc1.docx [retrieved on 2019-07-18]
- MARTIN DE BOCK ET AL: "Olive (Olea europaea L.) Leaf Polyphenols Improve Insulin Sensitivity in Middle-Aged Overweight Men: A Randomized, Placebo-Controlled, Crossover Trial", PLOS ONE, vol. 8, no. 3, 13 March 2013 (2013-03-13), page e57622, XP055265855, DOI: 10.1371/journal.pone.0057622 cited in the application
- POUDYAL HEMANT ET AL: "Hydroxytyrosol ameliorates metabolic, cardiovascular and liver changes in a rat model of diet-induced metabolic syndrome: Pharmacological and metabolism-based investigation", PHARMACOLOGICAL RESEARCH, ACADEMIC PRESS, LONDON, GB, vol. 117, 8 December 2016 (2016-12-08), pages 32-45, XP029921240, ISSN: 1043-6618, DOI: 10.1016/J.PHRS.2016.12.002 cited in the application
- YING SHEN ET AL: "Olive Leaf Extract Attenuates Obesity in High-Fat Diet-Fed Mice by Modulating the Expression of Molecules Involved in Adipogenesis and Thermogenesis", EVIDENCE-BASED COMPLEMENTARY AND ALTERNATIVE MEDICINE, 28 January 2014 (2014-01-28), pages 1-12, XP055281300, DOI: 10.1155/2014/971890
- Anonymous: "Enteric Coated Capsules 7.5 antioxidant and anti-inflammatory properties | FENÒLIA", , 1 January 2017 (2017-01-01), XP055684945, Retrieved from the Internet: URL:http://www.fenolia.it/en/enteric-coate d-capsules-hydroxytyrosol-from-olive-extra ct-antioxidant-and-anti-inflammatory-prope rties.php [retrieved on 2020-04-09]

## Description

The present invention concerns the use of olive fruit extract, in particular, in obese patients with a goal to reduce body-mass index and/or visceral fat. However, the present invention appears to be generally applicable when it comes to a treatment of overweight subjects regardless as to whether this treatment considers patients with severe conditions of overweight or generally subjects, such as mammals including human beings and animals which may benefit from any reduction in body-mass index and/or visceral fat.

The present invention is premised on the observation that the administration of an olive fruit extract comprising hydroxytyrosol (HT) in a significant amount reduces the body-mass index and/or visceral fat.

Hydroxytyrosol (HT) is a secoiridoid glycoside naturally occurring in olive fruit and olive oil. It was first discovered in the wastewater that resulted when green olives were rinsed in the process of making olive oil. Hydroxytyrosol is considered one of the most powerful scavengers of free radicals, reducing oxidative stress, flaccidity, age spots, and skin aging. It is also prevents protein damage induced by long-wave ultraviolet radiation in melanoma cells (D'Angelo S. et al. Free Radical Biology & Medicine 2005, 38, 908).

According to the Commission Regulation (EU) No 432/2012 on establishing a list of permitted health claims made on foods, olive oil polyphenols contribute to the protection of blood lipids from oxidative stress with a daily intake of at least 5 mg of hydroxytyrosol and its derivatives (e.g. oleuropein complex and tyrosol). These amounts, if provided by moderate amounts of olive oil, can be easily consumed in the context of a balanced diet (e.g. 20 g olive oil per day).

A clinical study in healthy volunteers revealed that the effect of a 3-week period consumption of 15 mg/day of hydroxytyrosol, could exert positive effects on human health reducing oxidative stress and cardiovascular risk, and improving lipid and plasma antioxidant profile, however, no significant changes in body composition parameters were observed (Colica C., et al. Oxidative Medicine and Cellular Longevity, 2017, Article ID 2473495, 14 pages).

A double blind randomized clinical study in 22 healthy subjects between 20 and 40 years old, who were treated with two different doses of hydroxytyrosol per day, in particular 5 mg and 25 mg and placebo for 7 days, showed no significant difference in the anthropometric variables of the subjects, body weight, fat and body mass index. Also, no changes observed in blood pressure and heartbeats of the examined subjects, nor any beneficial benefits in relation to their lipid profile. (Crespo M.S., et al., Pharmacological Research 95-96 (2015) 132-137).

Recent experiments in mice following a high-fat diet with current supplementation of oleuropein revealed the beneficial effects of oleuropein in reducing adiposity and resulted in levels of body weight, energy expenditure, serum lipids, hepatic lipid accumulation, adipocyte size and related gene expression profiles comparable to mice fed a normal-fat diet (Van der Stelt I., et al, Journal of Functional Foods 14 (2015) 702).

Another recent study in a diet-induced rat model of metabolic syndrome revealed that treatment with HT (20 mg/kg/d for 8 weeks) decreased adiposity, improved impaired glucose and insulin tolerance, improved endothelial function with lower systolic blood pressure, decreased left ventricular fibrosis and resultant diastolic stiffness and reduced markers of liver damage. These results were accompanied by reduced infiltration of monocytes/macrophages into the heart with reduced biomarkers of oxidative stress (Poudyal, H. et al, Pharmacological Research 117 (2017) 32).

US 2011/0142973 A1 discloses a method for use in treating a human suffering from obesity consisting essentially of administering to said human a therapeutically effective amount of an olive leaf extract to treat said obesity in the human.

A clinical study assessed the effect of hydroxytyrosol supplementation on body weight and showed that a 12-week supplementation of hydroxytyrosol (9.67 mg/day) associated with oleuropein (51.1 mg/day) did not exert any effect on body weight in overweight men (De Bock, M. et al, PLoS ONE 8 (2013) e57622).

A recent review article reports about the beneficial effects of hydroxytyrosol in the reduction of metabolic syndrome. However, no significant results in terms of weight and adiposity are reported (Peyro, J. et al, Nutrients 9 (2017) 306).

Although the antioxidant role of hydroxytyrosol is well established, however, there are confusing results regarding the effect of hydroxytyrosol consumption in body weight and visceral fat. In the same context, there is no study demonstrating a significant reduction in anthropometric parameters such as weight, waist circumference, weight-body index and visceral fat after consumption of specific amounts of hydroxytyrosol.

The present invention concerns a composition comprising hydroxytyrosol (HT). This composition can be derived from an extract of olive fruits (i.e. Olea Europaea from Greece). This composition is for the use in the treatment of overweight subjects. Such subjects may be either male or female. In particular, adult subjects over 21 of age are considered in the present invention.

In the context of the present invention, overweight is a medical condition in which excess body fat has accumulated to an extent that may have a negative effect on the health of the subject. A person is generally considered obese when its body-mass index (BMI), a measurement obtained by dividing a person's weight by the square of the person's height, is over 30 kg/m². Thus, a BMI of over 25-30 kg/m² is defined as overweight. Some East Asian countries, however, utilize lower values.

Obesity increases the likelihood of various diseases and conditions, particularly cardiovascular diseases, type 2 diabetes, obstructive sleep apnea, certain types of cancer, osteoarthritis, and depression.

Obesity is most commonly caused by a combination of excessive food intake, lack of physical activity, and genetic susceptibility.

Obesity is a major cause of death worldwide, with increasing rates in adults and children.

The use according to the invention treats the overweight subject with a daily dose of 15 to 20 mg HT. The administration may be carried out orally throughout the day and is typically effected before any intake of nutrition (i.e. breakfast, lunch, dinner). Preferably, a patient receives 15 mg HT per day.

The overweight person treated with the composition according to the invention may have at the beginning of the treatment a body-mass index of above 25 kg/m², or above 30 kg/m², or above 35 kg/m², or even above 40 kg/m².

Preferably, the overweight subject is a middle-aged woman (i.e. 30-50 years).

In another aspect, the present invention, while generally concerning the treatment of obesity, provides a means to prevent the side effects and conditions that are typically associated with obesity, such as cardiovascular diseases, type 2 diabetes, obstructive sleep apnea, certain types of cancer, or osteoarthritis and depression.

In another aspect, the present invention provides a capsule of a size in oval or oblong or round form with a volume of up to 2.50 ml, preferably from 0.20 to 1.20 ml, comprising an outer shell and 100 mg to 2000 mg of a liquid core composition comprising an extract of olive fruits, e.g. Olea Europaea. Such capsules may be used in the treatment of obesity to reduce the weight of a subject as outlined above. Such subject may be any type of mammal, including human beings, preferably female, and animals. The capsule may preferably comprise 200 mg to 1000 mg, and even more preferably 300 mg to 500 mg of the liquid core composition. The liquid core composition may in addition to the extract of olive fruits include additional excipients and/or active agents typical for capsule formulation, including edible oils.

The capsule may include in its liquid core composition preferably 2 mg to 10 mg hydroxytyrosol (HT), in particular 2.5 mg or 5 mg hydroxytyrosol.

According to a further aspect of the present invention, the invention concerns the non-therapeutic use of a nutrient or dietary supplement comprising or consisting of any composition as outlined above to reduce the body-mass index and/or visceral fat, e.g. of mammals. This use in particular concerns the non-medical application of the invention where during normal application of the supplement at no point in time any oversight by a medicinal practitioner is necessary or required.

A clinical study was undertaken in overweight subjects.

The olive fruit extract according to the invention and as utilized in this study is standardized in hydroxytyrosol, in total polyphenols content and in antioxidant activity. The assay of hydroxytyrosol in the olive fruit extract is determined with HPLC and ranges between 5 % and 15 % of the total mass of the olive fruit extract. The total polyphenols content in the olive fruit extract in this study is determined spectrophotometrically following the well-established Folin-Ciocalteu method and ranges between 100 mg and 400 mg of gallic acid per kilogram of the extract. The antioxidant activity of the olive fruit extract is evaluated in terms of IC₅₀ value of 2,2-diphenyl-1-picrylhydrazyl (DPPH) radical scavenging assay and determined between 80 and 100 µg/mL.

In any case, the final composition is standardized in the quantity of the olive fruit extract in order to provide a stable quantity of hydroxytyrosol at each time of administration, for example 2.5 mg or 5 mg hydroxytyrosol per capsule, or per 5 ml of a syrup.

The study is a randomized, double-blind, placebo-controlled clinical trial involving thirty (30) overweight women and aims to explore the effect of the consumption of the standardized in hydroxytyrosol and total polyphenols olive fruit extract (15 mg of hydroxytyrosol daily) for a period of six months.

The standardized olive fruit extract in this study is encapsulated in soft-gelatin capsules, where each capsule comprises 2.5 mg of hydroxytyrosol and of 5 mg of total polyphenols, further comprising lecithin as emulsifier and olive oil as lipid carrier. Placebo capsules were formed in the same way as above but in the absence of the standardized olive fruit extract.

The subjects were divided in three groups as follows:
**Group A:** 10 subjects consuming 2 capsules with the standardized olive fruit extract before the three main lunches, daily (15 mg hydroxytyrosol and 30 mg total polyphenols in total).
**Group B:** 10 subjects consuming 2 capsules with the standardized olive fruit extract and 4 capsules with placebo before the three main lunches, daily (5 mg hydroxytyrosol and 10 mg total polyphenols in total).
**Group C:** 10 subjects consuming 6 capsules with placebo before the three main lunches daily.

The groups are regulated in terms of age, body-mass index and waist circumference. All participants in this study authorized their participation by reading and signing an informed consent with the provisions of this study.

The inclusion criteria are as follows:
- The subject has to be an adult female with a body-mass index between 27 kg/m² and 35 kg/m² and a waist circumference of up to 130 cm.
- The subject may have a stable body weight up to 3 months before the beginning of the study (variance ± 5%).
- The subject has to be under a regular hypolipidemic medical treatment before entering the study.

The exclusion criteria include neoplastic, autoimmune, liver and kidney diseases, type 1 and type 2 diabetes, heart failure, liver dysfunction, pregnancy, non-regulated hypo- or hyperthyroidism, medical treatment against obesity.

The anthropometric parameters (body weight, fat mass and visceral fat) of the subjects are monitored and recorded in the beginning of the study and then in the 1st, 3rd and 6th month of the study. Seven of the thirty subjects enrolled in the study are excluded due to no compliance (2 from Group A, 1 from Group B and 4 from Group C). No side effects to the subjects, related to HT consumption, are observed. The results are summarized in the following Tables.

**Table 1: Average body weight loss during clinical study**

| **Time Interval** | **Average Body Weight Loss** | | |
|---|---|---|---|
| | **Group A** | **Group B** | **Group C** |
| 1^{st} month (*p* = 0.01) | - 4.17 Kg | - 1.27 Kg | - 1.98 Kg |
| 3^{rd} month (*p* = 0.005) | - 8.00 Kg | - 2.77 Kg | - 4.00 Kg |
| 6^{th} month (*p* < 0.001) | - 11.44 Kg | - 2.73 Kg | - 2.33 Kg |

**Table 2: Average Fat Mass Loss during clinical study**

| **Time Interval** | **Average Fat Mass Loss** | | |
|---|---|---|---|
| | **Group A** | **Group B** | **Group C** |
| 1^{st} month (*p* = 0.08) | - 3.20 Kg | - 1.10 Kg | - 2.46 Kg |
| 3^{rd} month (*p* = 0.0017) | - 6.30 Kg | - 2.67 Kg | - 2.58 Kg |
| 6^{th} month (*p* = 0.00) | - 9.55 Kg | - 2.06 Kg | - 2.13 Kg |

**Table 3: Average Visceral Fat Loss during clinical study**

| **Time Interval** | **Average Visceral Fat Loss** | | |
|---|---|---|---|
| | **Group A** | **Group B** | **Group C** |
| 1^{st} month (*p* = 0.01) | - 1.87 Kg | - 0.22 Kg | - 0.58 Kg |
| 3^{rd} month (*p* = 0,00) | - 3.06 Kg | - 0.62 Kg | - 1.33 Kg |
| 6^{th} month (*p* = 0,00) | - 3.85 Kg | - 0.56 Kg | - 1.87 Kg |

It has been surprisingly found that the consumption of 15 mg hydroxytyrosol on a daily base for six months (Group A) presents a beneficial effect in the body weight and therefore in the body-mass index reduction, furthermore, in the fat mass and the visceral fat of overweight women. It is noteworthy to mention that this effect is noticeable from the first month and proceeds increasingly up to the 6^{th} month for the studied Group A. On the contrary, the consumption of 5 mg hydroxytyrosol on a daily base for Group B does not show any significant effect in the average weight and consequently to any of the above mentioned anthropometric parameters. In addition, no significant differences between the results of Group B (5 mg hydroxytyrosol group) and Group C (placebo group) are observed.

Overall, a regular intake of a composition comprising a standardized in hydroxytyrosol and total polyphenols olive fruit extract, which ensures the consumption of 15 mg hydroxytyrosol and 60 mg in total polyphenols per day, can be an effective treatment in reducing the body-mass index and the visceral fat of overweight human subjects.

The olive fruit extract, which is standardized in hydroxytyrosol and total polyphenol content can be formulated in either solid or liquid compositions of several strengths following standard procedures. In a preferred embodiment the solid compositions, which are suitable for oral administration, comprise of the standardized olive fruit extract and at least one pharmaceutically acceptable excipient including polysaccharides, starch and cellulose. Solid compositions of the standardized olive fruit extract can be formulated in various forms, including tablets, lozenges, powders or granules. In a preferred embodiment, the solid composition comprising of the standardized olive fruit extract is encapsulated in hard capsules.

In a preferred embodiment, the standardized olive fruit extract is dispersed in one or more pharmaceutically acceptable liquid excipients like an edible oil among of olive oil, sesame oil, sunflower oil, palm oil, peanut oil, cottonseed oil, corn oil, coconut oil, soybean oil, and safflower oil. The liquid composition comprising the standardized olive fruit extract is encapsulated in soft capsules of gelatin or vegetable origin or is administered in the form of a syrup or elixir.

## Claims

1. A pharmaceutical composition, derivable from an extract of olive fruits (Olea Europaea) comprising hydroxytyrosol (HT), for use in the treatment of overweight subjects, having a body-mass index of above 25 kg/m², in order to reduce the body-mass index, fat mass and visceral fat, wherein the subject receives a daily dose between 15 and 20 mg HT.

2. The pharmaceutical composition for the use according to claim 1, wherein the subject is female, preferably a middle-aged (30-50 years) woman.

3. The pharmaceutical composition for the use according to claim 1 or 2, which is in the form of tablets, lozenges, powders or granules, or encapsulated in hard capsules, or encapsulated in soft capsules of gelatin or vegetable origin, or is administered in the form of a syrup or elixir.

4. The pharmaceutical composition for the use according to any one of claims 1 to 3, wherein the daily dose of HT is 15 mg and is provided for 1 month, 3 months or 6 months.

5. A soft capsule in oval or oblong or round form with a volume of up to 2.50 ml, comprising an outer shell and 100 mg to 2000 mg of a liquid core composition, comprising a pharmaceutical composition, derivable from the extract of olive fruits (Olea Europaea) comprising hydroxytyrosol (HT), for use in the treatment of overweight subjects, having a body-mass index of above 25 kg/m², .in order to reduce the body-mass index, fat mass and visceral fat, wherein the subject receives a daily dose between 15 and 20 mg HT.

6. The capsule for the use according to claim 5, comprising 300 mg to 500 mg of a liquid core composition.

7. The capsule for the use according to claim 5 or 6, wherein the daily dose of HT is 15 mg and is provided for 1 month, 3 months or 6 months.

8. Non-therapeutic use of a nutrition or dietary supplement for human beings having a body-mass index of above 25 kg/m², comprising a composition according to any one of the preceding claims 1 to 4 or a capsule according to claims 5 to 7, to reduce the body-mass index, fat mass and visceral fat, wherein the human being receives a daily dose between 15 and 20 mg HT.

## Patentansprüche

1. Pharmazeutische Zusammensetzung, die aus einem Extrakt von Olivenfrüchten (Olea Europaea) erhalten werden kann, umfassend Hydroxytyrosol (HT), zur Verwendung bei der Behandlung übergewichtiger Subjekte mit einem Body-Mass-Index von über 25 kg/m², um den Body-Mass-Index, die Fettmasse und das viszerale Fett zu reduzieren, wobei das Subjekt eine tägliche Dosis zwischen 15 und 20 mg HT erhält.

2. Die pharmazeutische Zusammensetzung zur Verwendung gemäß Anspruch 1, wobei das Subjekt weiblich ist, bevorzugt eine Frau mittleren Alters (30-50 Jahre).

3. Die pharmazeutische Zusammensetzung zur Verwendung gemäß Anspruch 1 oder 2, die in Form von Tabletten, Lutschtabletten, Pulver oder Granulat oder eingekapselt in Hartkapseln oder eingekapselt in Weichkapseln aus Gelatine oder pflanzlicher Herkunft vorliegt, oder in Form eines Sirups oder Elixiers verabreicht wird.

4. Die pharmazeutische Zusammensetzung zur Verwendung gemäß einem der Ansprüche 1 bis 3, wobei die Tagesdosis von HT 15 mg beträgt und für 1 Monat, 3 Monate oder 6 Monate bereitgestellt wird.

5. Weichkapsel in ovaler oder länglicher oder runder Form mit einem Volumen von bis zu 2,50 ml, umfassend eine äußere Hülle und 100 mg bis 2000 mg einer flüssigen Kernzusammensetzung, die eine pharmazeutische Zusammensetzung umfasst, die aus dem Extrakt von Olivenfrüchten (Olea Europaea) abgeleitet werden kann, umfassend Hydroxytyrosol (HT), zur Verwendung bei der Behandlung von übergewichtigen Subjekten mit einem Body-Mass-Index von über 25 kg/m², .um den Body-Mass-Index, die Fettmasse und das viszerale Fett zu reduzieren, wobei das Subjekt eine tägliche Dosis zwischen 15 und 20 mg HT erhält.

6. Die Kapsel zur Verwendung nach Anspruch 5, umfassend 300 mg bis 500 mg einer flüssigen Kernzusammensetzung.

7. Die Kapsel zur Verwendung gemäß Anspruch 5 oder 6, wobei die Tagesdosis von HT 15 mg beträgt und für 1 Monat, 3 Monate oder 6 Monate bereitgestellt wird.

8. Nicht-therapeutische Verwendung eines Nahrungs- oder Diätergänzungsmittels für Menschen mit einem Body-Mass-Index von über 25 kg/m², umfassend eine Zusammensetzung gemäß einem der vorhergehenden Ansprüche 1 bis 4 oder eine Kapsel gemäß den Ansprüchen 5 bis 7, um den Body-Mass-Index, die Fettmasse und das viszerale Fett zu reduzieren, wobei der Mensch eine Tagesdosis zwischen 15 und 20 mg HT erhält.

## Revendications

1. Composition pharmaceutique, pouvant être dérivée d'un extrait d'olives (Olea Europaea) comprenant de l'hydroxytyrosol (HT), pour une utilisation dans le traitement de sujets en surpoids, ayant un indice de masse corporelle supérieur à 25 kg/m², afin de réduire l'indice de masse corporelle, la masse grasse et la graisse viscérale, le sujet recevant une dose quotidienne comprise entre 15 et 20 mg d'HT.

2. Composition pharmaceutique pour l'utilisation selon la revendication 1, dans laquelle le sujet est une femme, de préférence une femme d'âge moyen (30 à 50 ans).

3. Composition pharmaceutique pour l'utilisation selon la revendication 1 ou 2, qui se présente sous forme de comprimés, de pastilles, de poudres ou de granulés, ou encapsulée dans des gélules, ou encapsulée dans des capsules molles de gélatine ou d'origine végétale, ou est administrée sous la forme d'un sirop ou élixir.

4. Composition pharmaceutique pour l'utilisation selon l'une quelconque des revendications 1 à 3, dans laquelle la dose quotidienne d'HT est de 15 mg et est fournie pendant 1 mois, 3 mois ou 6 mois.

5. Capsule molle de forme ovale ou oblongue ou ronde d'un volume allant jusqu'à 2,50 ml, comprenant une enveloppe externe et 100 mg à 2 000 mg d'une composition de noyau liquide, comprenant une composition pharmaceutique, pouvant être dérivée de l'extrait d'olives (Olea Europaea) comprenant de l'hydroxytyrosol (HT), pour une utilisation dans le traitement de sujets en surpoids, ayant un indice de masse corporelle supérieur à 25 kg/m², afin de réduire l'indice de masse corporelle, la masse grasse et la graisse viscérale, dans laquelle le sujet reçoit une dose quotidienne comprise entre 15 et 20 mg d'HT.

6. Capsule pour l'utilisation selon la revendication 5, comprenant 300 mg à 500 mg d'une composition de noyau liquide.

7. Capsule pour l'utilisation selon la revendication 5 ou 6, dans laquelle la dose quotidienne d'HT est de 15 mg et est fournie pendant 1 mois, 3 mois ou 6 mois.

8. Utilisation non thérapeutique d'un aliment ou complément alimentaire pour être humain ayant un indice de masse corporelle supérieur à 25 kg/m², comprenant une composition selon l'une quelconque des revendications 1 à 4 précédentes ou une capsule selon les revendications 5 à 7, pour réduire l'indice de masse corporelle, la masse grasse et la graisse viscérale, dans laquelle l'être humain reçoit une dose quotidienne comprise entre 15 et 20 mg d'HT.
